# EUROPEAN PATENT APPLICATION

(11) **EP 3 854 354 A1**
(43) Date of publication of application: **28.07.2021**
(21) Application number: 19945239.2
(22) Date of filing: 11.09.2019
(51) Int. Cl.: A61F 2/38, A61F 2/30

(54) **ARTIFICIAL KNEE JOINT**

(71) Applicant: Corentec Co., Ltd., Cheonan-si, Chungcheongnam-do 31056 (KR)
(72) Inventor: JANG, Young Woong, Seoul 06122 (KR); KIM, Chan Eol, Seoul 02801 (KR); RO, Jae Hun, Seoul 05571 (KR); HAN, Ah Reum, Chuncheon-si, Gangwon-do 24318 (KR); LEE, Myung Chul, Seongnam-si, Gyeonggi-do 13455 (KR); IN, Young, Seoul 06215 (KR); HAN, Seung Beom, Seoul 03010 (KR)
(74) Representative: Witte, Weller & Partner Patentanwälte mbB
(86) International application number: PCT/KR2019/011882
(87) International publication number: WO 2021/049690

(57) **Abstract**

The present disclosure relates to an artificial knee joint and, more particularly, to an artificial knee joint comprising a tibial element implanted at the proximal end of the tibia and a bearing element coupled to the tibial element, wherein the tibial element includes an interference preventing portion capable of preventing interference with the bearing element when the bearing element is inserted in an oblique direction, and the bearing element includes a protrusion coupled to the interference preventing portion.

## Description

### Technical Field

The present disclosure relates to an artificial knee joint and, more particularly, to an artificial knee joint including a tibial element implanted at the proximal end of the tibia and a bearing element coupled to the tibial element, wherein the tibial element includes an interference preventing portion capable of preventing interference with the bearing element when the bearing element is inserted in an oblique direction, and the bearing element includes a protrusion coupled to the interference preventing portion.

### Background Art

Among many joints that make up the body, the knee joint is a joint part that connects the tibia and the femur, and the number of patients who are in an unrecoverable state due to wear of the knee joint, aging of bone tissue, accidents, and the like has been gradually increased. The knee joint is located between the lower end of the femur, the upper end of the tibia, and the back side of the patella (kneecap), and functions to bend the leg from the knee to the back.

Recently, an artificial knee replacement procedure has been widely performed in patients who cannot recover due to severe damage to the joint area, and metal, ceramic, or polyethylene is used for the joint motion part of such an artificial knee joint, which has excellent mechanical properties, has a small coefficient of friction, and increases biocompatibility. In general, the artificial knee joint is divided into a femur insertion member coupled to the distal end of the femur, a tibial element coupled to the distal end of the tibia, and a bearing element (part corresponding to the cartilage) positioned between the femur insertion member and the tibial element. Here, the femur insertion member and the tibial element are mainly made of a metal alloy, and the bearing element is made of polyethylene or the like.

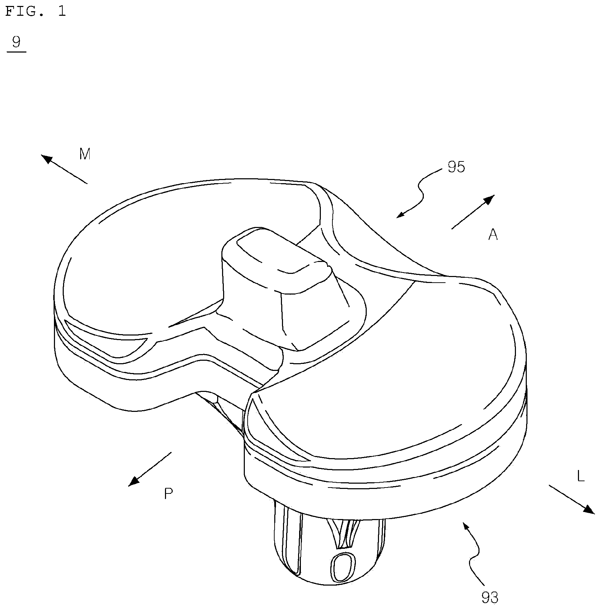

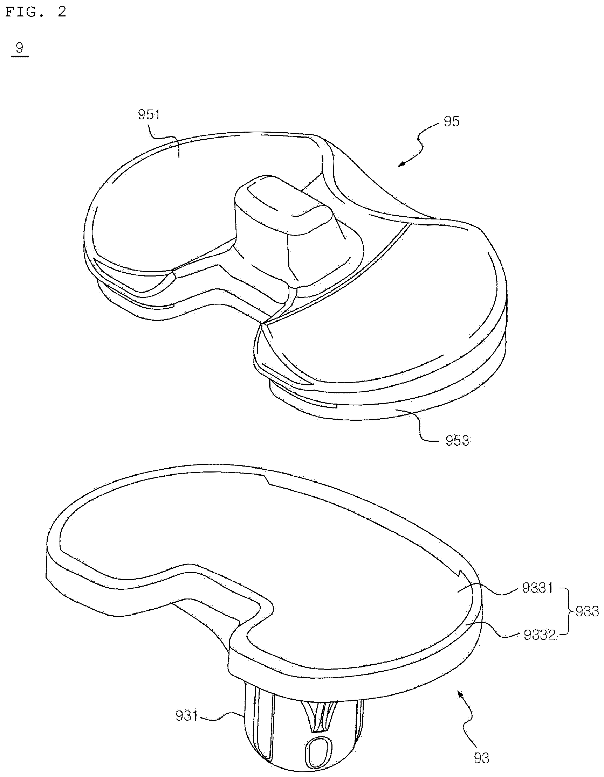

FIG. 1 is a perspective view showing a conventional artificial knee joint, and FIG. 2 is an exploded perspective view of FIG. 1, which is disclosed in Korean Patent Registration No. 10-1352066. Referring to FIGS. 1 and 2, the artificial knee joint 9 includes a bearing element 95 that is coupled to the upper side of a tibial element 93 to replace the cartilage of the actual knee joint, and a tibial element 93 that is inserted into the upper side of the tibia and receives the bearing element 95 on the upper side to couple with the bearing element 95.

The bearing element 95 includes an upper portion 951 that is in contact with a femoral element not shown and provides an articular surface for joint motion, and a lower portion 953 that extends downwardly from the lower surface of the upper portion through a stepped jaw and is coupled to the tibial element 93.

In addition, the tibial element 93 includes a base plate 933 that defines a coupling space into which the lower portion of the bearing element is inserted, and a stem 931 that extends from a lower side of the base plate.

The base plate 933 includes a plate bottom 9331 and a rim 9332 that extends upwardly along an outer periphery of the plate bottom.

The base plate 933 of the tibial element 93 and the lower portion 953 of the bearing element 95 have a corresponding shape and structure so as to be coupled to each other. Such a configuration is disclosed in Korean Patent Registration No. 10-1352066.

In the process of performing the artificial knee joint 9 procedure, when combining the bearing element 95 with the tibial element 93, it is important that the correct position is aligned and the coupling is made in the correct position. This is because, if the coupling is not made in the correct position, a problem may occur in that the bearing element 95 may be damaged or the tibial element 93 may be separated from its position.

Meanwhile, minimally invasive knee replacement surgery that minimizes the skin incision site is desirable for patients. The advantages of the minimally invasive knee replacement surgery includes less blood loss during surgery and less post-operative pain as well as less cosmetic problems by minimizing incision and exfoliation of the skin and surrounding soft tissue for surgery, and a short recovery period and quick rehabilitation treatment because muscle ligaments are not cut. However, in the process of knee replacement surgery that minimizes the skin incision sites, there are various restrictions on the procedure. That is, when the skin is incised, in a case in which the skin is incised inward, the patella is tilted outward, and the tibial element 93 is inserted and then finally combined with the bearing element 95, the bearing element 95 must be inserted in an oblique direction due to the surrounding tissue.

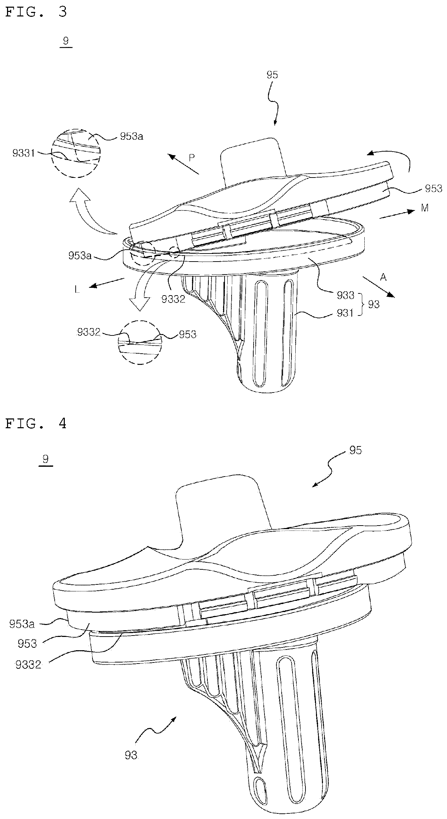

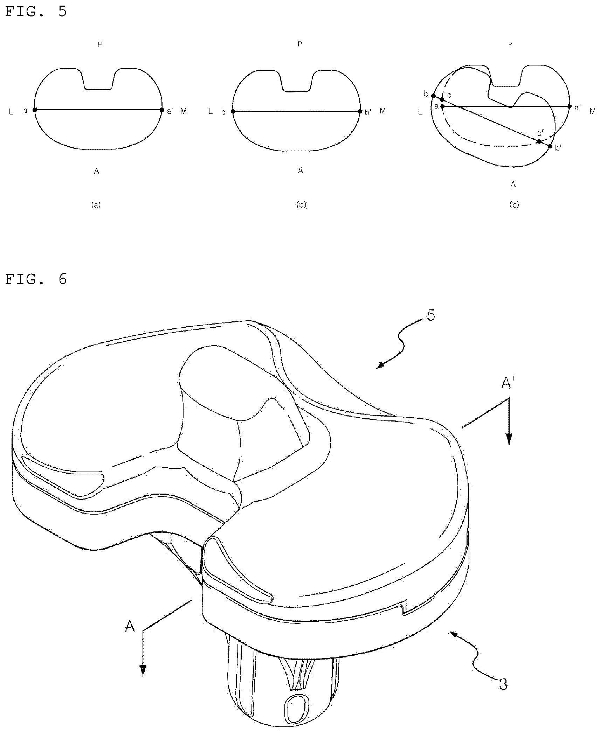

FIG. 3 is a view showing a state of use of an artificial knee joint, which is a prior art, and is a view showing a case of inserting a bearing element into a tibial element in an oblique direction. FIG. 4 is a view showing a case in which interference occurs in the process of inserting a bearing element into a tibial element in an oblique direction, subsequent to FIG. 3. FIG. 5A is a view showing a case in which a tibial element is projected onto a plane from the top side, FIG. 5B is a view showing a case in which a bearing element is projected onto a plane from the top side, and FIG. 5C is a view showing a case in which a state where a bearing element is inserted into a tibial element in an oblique direction is projected onto a plane from the top side.

As shown in FIG. 3, when the procedure is performed in the oblique direction, the surgeon moves the bearing element 95 in the oblique direction toward the tibial element 93 implanted at the proximal end of the tibia, and then places a left or right posterior end 953a in the middle of the lower portion 953 of the bearing element 95 in the inner space of the rim 9332 of the corresponding base plate 933, that is, in the left or right space of the space. However, as shown in FIG. 5, since the length of a segment a-a' of a major axis, which is the longest axis in a coupling space inside the rim 9332 of the base plate 933, and the length of a segment b-b' of a major axis, which is the longest axis in the lower portion of an insert, are approximately the same, when the bearing element 95 is inserted into the tibial element 93 in the oblique direction, the segment b-b' of the major axis of the lower portion of the bearing element 95 is located at a position other than the segment a-a' of the major axis of the base plate, so that a part of the lower portion 953 of the bearing element 95 collides with the upper end of the rim 9332 of the base plate 933 as shown in FIG. 3, and the left or right posterior end 953a in the middle of the lower portion 953 of the bearing element 95 that has already been inserted is lifted upward as shown in FIG. 4, whereby the insertion in the oblique direction becomes difficult. Referring to FIG. 5C, the segment b-b' of the major axis of the lower portion of the bearing element is longer than a segment c-c' obtained by connecting points where the major axis of the lower portion of the insert meets an inner end of an upper surface of the rim of the base plate, whereby the insertion in the oblique direction becomes difficult.

### Detailed Description of the Invention

### Technical Problem

Therefore, the present disclosure has been made in view of the above-mentioned problems.

An aspect of the present disclosure is to provide an artificial knee joint that includes an interference preventing unit to prevent interference between a bearing element and a tibial element when the bearing element is inserted into the tibial element in an oblique direction, thereby facilitating insertion in the oblique direction.

Another aspect of the present disclosure is to provide an artificial knee joint that includes a protrusion having a shape complementary to the interference preventing portion and coupled to the interference preventing portion, thereby facilitating alignment of the coupling position of the tibial element and the bearing element.

Still another aspect of the present disclosure is to provide an artificial knee joint that combines the protrusion of the bearing element and a recessed groove of the tibial element each having a complementary shape, thereby preventing rotation of the bearing element due to movement of the artificial knee joint and preserving the position of the bearing element.

Yet another aspect of the present disclosure is to provide an artificial knee joint in which a connection between the protrusion and the recessed groove is made without a gap, thereby preventing side effects such as the surrounding tissues being pinched in a coupling gap in a recovery process after the procedure is completed.

### Technical Solution

The present disclosure is implemented by an embodiment having the following configuration in order to achieve the above objects.

According to an embodiment of the present disclosure, an artificial knee joint may include: a tibial element implanted at a proximal end of the tibia, and a bearing element coupled to the tibial element, wherein the tibial element includes an interference preventing portion capable of preventing interference with the bearing element when the bearing element is inserted in an oblique direction.

According to another embodiment of the present disclosure, the tibial element may include a base plate coupled to the bearing element, and the interference preventing portion may be formed on the base plate.

According to another embodiment of the present disclosure, the interference preventing portion may be formed on an outer periphery of the tibial element.

According to another embodiment of the present disclosure, the interference preventing portion may be a recessed groove formed by being recessed at a predetermined position of the outer periphery of the tibial element.

According to another embodiment of the present disclosure, the base plate may include a rim protruding along an outer periphery thereof, and the recessed groove may be formed in the rim.

According to another embodiment of the present disclosure, the interference preventing portion may be formed symmetrically with respect to line A-P.

According to another embodiment of the present disclosure, the interference preventing portion may include one end and the other end, and one end may be located at a posterior side and the other end may be located at an anterior side with respect to line M-L.

According to another embodiment of the present disclosure, the bearing element may include a protrusion coupled to the interference preventing portion.

According to another embodiment of the present disclosure, the protrusion may have a shape complementary to the interference preventing portion.

### Advantageous Effects

The present disclosure can obtain the following effects by the configuration, combination, and use relationship that will be described below with the present embodiment.

The artificial knee joint according to the present disclosure may include an interference preventing portion to prevent interference between the bearing element and the tibial element when the bearing element is inserted into the tibial element in the oblique direction, thereby facilitating insertion in the oblique direction.

The artificial knee joint according to the present disclosure may include a protrusion having a shape complementary to the interference preventing portion and coupled to the interference preventing portion, thereby facilitating alignment of the coupling position of the tibial element and the bearing element.

The artificial knee joint according to the present disclosure may combine the protrusion of the bearing element and the recessed groove of the tibial element each having a complementary shape, thereby preventing rotation of the bearing element due to movement of the artificial knee joint and preserving the position of the bearing element.

The artificial knee joint according to the present disclosure may combine the protrusion and the recessed groove without a gap therebetween, thereby preventing side effects such as pinching of the surrounding tissue in a coupling gap in a recovery process after the procedure is finished.

### Brief Description of the Drawings

FIG.1 is a perspective view showing a conventional artificial knee joint.

FIG. 2 is an exploded perspective view of FIG. 1.

FIG. 3 is a view showing a state of use of an artificial knee joint, which is a prior art, and is a view showing a case of inserting a bearing element into a tibial element in an oblique direction.

FIG. 4 is a view showing a case in which interference occurs in the process of inserting a bearing element into a tibial element in an oblique direction, subsequent to FIG. 3.

FIG. 5A is a view showing a case in which a tibial element is projected onto a plane from the top side, FIG. 5B is a view showing a case in which a bearing element is projected onto a plane from the top side, and FIG. 5C is a view showing a case in which a state where a bearing element is inserted into a tibial element in an oblique direction is projected onto a plane from the top side.

FIG. 6 is a perspective view showing an artificial knee joint according to an embodiment of the present disclosure.

FIG. 7 is an exploded perspective view of FIG. 6.

FIG. 8 is a plan view showing a tibial element according to an embodiment of the present disclosure.

FIG. 9 is a lower perspective view showing a bearing element according to an embodiment of the present disclosure.

FIG. 10 is a cross-sectional view taken along line A-A' of FIG. 6.

FIG. 11 is a view showing a case in which interference does not occur when a bearing element is inserted into a tibial element in an oblique direction, as a state of use of the present disclosure.

FIG. 12 is a view showing a case in which smooth coupling is performed without interference in the process of inserting a bearing element into a tibial element in an oblique direction and coupling to each other, subsequent to FIG. 11.

### Mode for Carrying Out the Invention

Hereinafter, an artificial knee joint according to the present disclosure will be described in detail with reference to the accompanying drawings. It should be noted that the same components in the drawings are indicated by the same reference numerals wherever possible. In addition, detailed descriptions of known functions and configurations that may unnecessarily obscure the subject matter of the present disclosure will be omitted. Unless otherwise defined, all terms in this specification are the same as the general meaning of the terms understood by those of ordinary skill in the art to which the present disclosure belongs. In case of conflict with the meaning of the terms used in the present specification, the definitions used in the present specification are applied.

In the present specification, with respect to a coronal plane, A refers to an anterior side in a direction in which a person's face faces and P refers to a posterior side in a direction in which the posterior side of a person's head faces, and M means medial and L means lateral with respect to a sagittal plane.

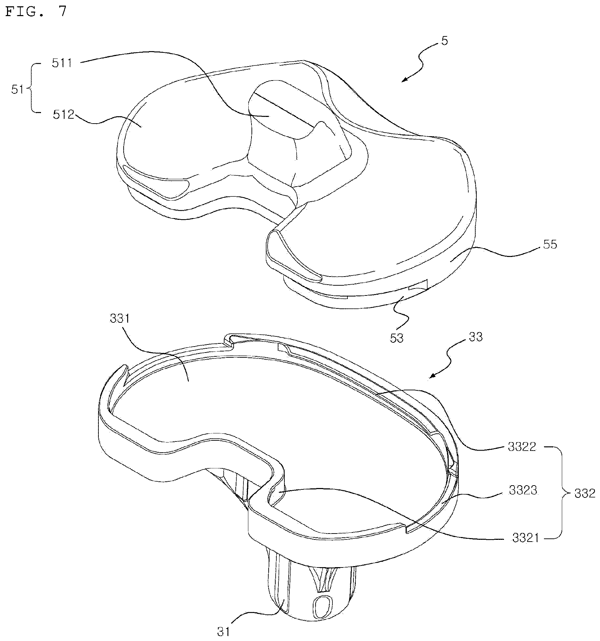

FIG. 6 is a perspective view showing an artificial knee joint according to an embodiment of the present disclosure, and FIG. 7 is an exploded perspective view of FIG. 6. Referring to FIGS. 6 and 7,

The artificial knee joint according to an embodiment of the present disclosure includes a tibial element 3 coupled to the tibia end and a bearing element 5 coupled to the upper side of the tibial element, and a femur insertion member (not shown) slides on the upper surface of the bearing element 5 and performs a joint motion similar to an actual knee joint while performing a flexion or rotational motion, thereby functioning as an artificial joint.

The tibial element 3 is inserted into the upper side of the tibia, and includes a stem 31 and a base plate 33, as a configuration formed in a predetermined shape constituting a part of the tibial element by accommodating the bearing element 5 on the upper side of the tibial element 3 and combining with the bearing element 5.

The stem 31 is a portion that is embedded in the patient's tibia in the procedure of an artificial knee joint, and may be largely divided into a bone cement fixed stem for improving the bonding strength between the tibia and the stem 31 and a cementless stem that does not use bone cement, and may have various structures for internal fixation of the tibia of the stem 31.

The base plate 33 is a portion where the bearing element 5 is seated, and includes a plate bottom 331 and a rim 332, and may be usually made of a single metal material such as Ti alloy or CoCr alloy, but the material of the plate bottom 331 and the rim 332 can be manufactured differently when lamination technology is used.

The plate bottom 331 is a portion on which the rim 332 is erected and where a bonding surface between the base plate 33 and the stem 31 is present below the rim 332, and the base plate 33 and the stem 31 are combined with each other in a manner that the lower portion of the plate bottom 331 and the upper surface of the stem are joined to each other.

The rim 332 is formed to protrude upward along the outer periphery of the bottom of the plate and is erected on the plate bottom 531 to define a coupling space in which the bearing element 5 of the artificial knee joint is seated. The rim 332 may be customized to a patient by having different shapes, heights, and sizes depending on the patient, and includes a posterior protrusion 3321, an anterior groove 3322, and a recessed groove 3323.

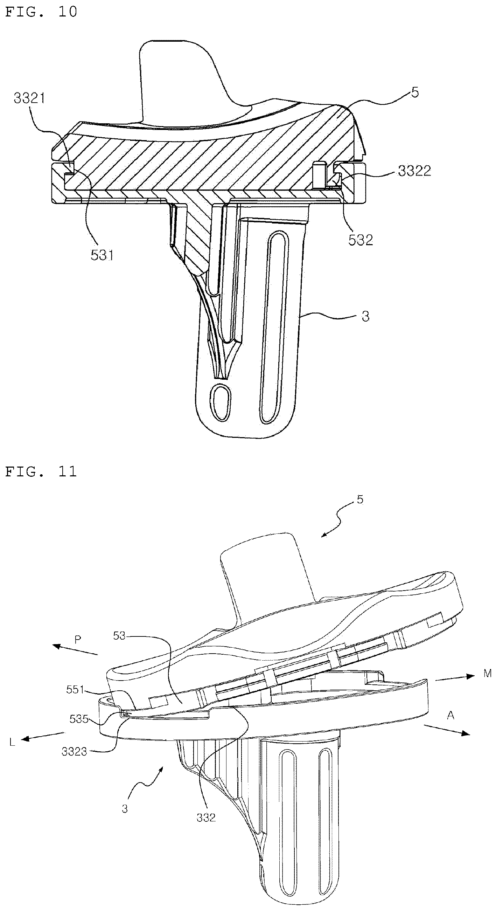

The posterior protrusion 3321 is a portion formed by bending and extending from the rim 332 in the horizontal direction, and is inserted into a coupling groove 531 of the bearing element 5 so that a coupling therebetween is made. Such a coupling relationship can be seen in FIG. 10.

The anterior groove 3322 is a portion generated in the process of bending and extending the posterior protrusion 3321 in the horizontal direction, and has a " 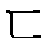 " shape in cross section, and an elastic hook 532 of the bearing element 5 is inserted into the anterior groove 3322 so that an elastic coupling therebetween is made. Such a coupling relationship can be seen in FIG. 10.

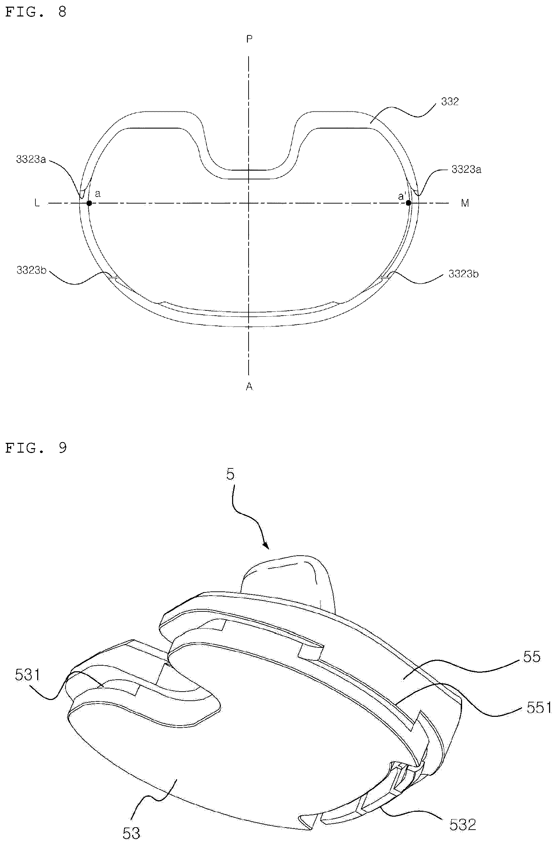

As shown in FIG. 8, the recessed groove 3323 is a portion recessed downward from the rim 332 by a certain depth, and has one end 3323a at which the recession begins and the other end 3323b at which the recession ends. One end 3323a is a portion close to the posterior end of the base plate 33, and the other end 3323b is a portion close to the anterior end of the base plate. Here, the posterior end is a portion facing a posterior (P) side P from the base plate, and the anterior end is a portion facing an anterior side A from the base plate. The distance and position between the one end 3323a and the other end 3323b may be configured in various ways. However, in order to insert the bearing element in the oblique direction, when a lateral (L) or medial (M) posterior end 535 in the middle of the lower portion of an inserted insert is located in a corresponding coupling space of the base plate 33, that is, an outer or inner space of the space, the lower portion of the bearing element located on the anterior side of the posterior end is formed so as not to collide with the inner end of the upper surface of the rim of the base plate. Preferably, with respect to line M-L, which is a medial and lateral line, one end 3323a is formed to be located behind line M-L and the other end 3323b is formed to be located in front of line M-L.

According to another embodiment of the present disclosure, the recessed grooves 3323 are formed in pairs to be symmetrical to each other on the medial (M) and lateral (L) side of the rim of the base plate. In addition, it is not excluded that the gap or position of the recessed grooves may be formed asymmetrically. For example, the recessed groove of the medial (M) side and the recessed groove of the lateral (L) side are formed at different intervals, or the interval of the recessed grooves located in the insertion direction should be made longer than that of the recessed grooves located in the opposite direction.

FIG. 9 is a lower perspective view showing a bearing element according to an embodiment of the present disclosure. Referring to FIGS. 7 and 9,

The bearing element 5 is located between the femur insertion member and the tibial element 3 and is a configuration that replaces the cartilage of the actual knee joint. The bearing element 5 is coupled to the upper side of the tibial element 5, and includes an upper portion 51, a lower portion 53, and a side portion 55.

Referring to FIG. 7, the upper portion 51 includes a post 511 protruding upward from the bearing element 5, and a groove 512 that extends to the lower end of the post 511 and provides an articular surface of the femur insertion member (not shown) and an articular surface for joint motion.

The post 511 is a configuration that protrudes upward on one side of the upper surface of the bearing element 5. There are a cruciate retaining (CR) type artificial knee joint procedure and a posterior stabilized (PS) type artificial knee joint procedure. The CR type is a type of the artificial knee joint for performing an artificial knee joint procedure without removing the posterior cruciate ligament, and the PS type is a type of the artificial knee joint that replaces the posterior cruciate ligament with the post 511 of the bearing element 5 in the state where the posterior cruciate ligament is removed. In the case of the PS type artificial knee joint, it replaces the posterior cruciate ligament removed by the post 511 of the bearing element 5 and a cam of the femur insertion member. Therefore, the post 511 is a configuration necessary for an artificial knee joint procedure when the posterior cruciate ligament is removed and is not an essential configuration of the present disclosure, and may be included in the bearing element 5 according to the procedure.

The groove 512 is a portion that extends to the lower end of the post 511 and provides an articular surface that abuts on an articular surface of the femur insertion member (not shown) to perform joint motion.

Referring to FIG. 9, the lower portion 53 extends downwardly through a stepped jaw from the lower side of the upper portion and includes a coupling groove 531 and an elastic hook 532 for coupling with the tibial element 3.

The coupling groove 531 is a portion that is recessed on the posterior surface of the bearing element 5, that is, in a posterior (P) direction, and when combined with the tibial element 3, the posterior protrusion 3321 of the tibial element 3 is inserted into the coupling groove 531 to achieve a solid coupling. Such a coupling relationship can be seen in FIG. 10.

The elastic hook 532 is a portion protruding from the anterior surface of the bearing element, that is, in the anterior (A) direction, and when combined with the tibial element 3, the elastic hook 532 is inserted into an anterior groove 5322 of the tibial element 3 to achieve a solid coupling. Such a coupling relationship can be seen in FIG. 10.

The side portion 55 is a portion that connects the upper portion 31 and the lower portion 53 and includes a protrusion 551 as a portion forming a circumference of the bearing element 5.

The protrusion 551 is a portion that protrudes downwardly from the side portion 55 of the bearing element 5 and has a shape complementary to the recessed groove 3323 of the tibial element 3. In the process of performing minimally invasive knee replacement surgery as described above, a case may occur in which the bearing element 5 is inserted into the tibial element 3 in the oblique direction due to the restriction caused by minimization of the skin incision site, and then the coupled position is aligned. Here, the present disclosure has the effect of allowing the recessed groove 3323 of the tibial element to receive the protrusion 551 to guide the coupling at the correct position. In addition, since the protrusion 551 of the bearing element 5 and the recessed groove 3323 of the tibial element 3 having a complementary shape are combined with each other, rotation of the bearing element 5 due to the motion of the artificial knee joint may be prevented to preserve the position of the bearing element 5. Further, since the coupling between the protrusion 551 and the recessed groove 3323 is made without a gap, side effects such as the surrounding tissues being pinched in the coupling gap in the recovery process after the procedure is completed can be prevented.

FIG. 11 is a view showing a case in which interference does not occur when a bearing element is inserted into a tibial element in an oblique direction, as a state of use of the present disclosure.

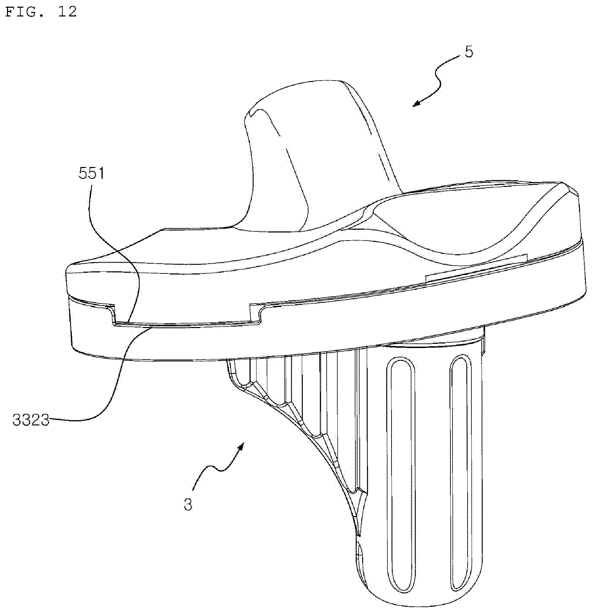

Referring to FIG. 11, as described above, in the procedure for minimizing the skin incision site, the bearing element is inserted in an oblique direction from the anterior (A) side to the posterior (P) side and the lateral (L) side due to the surrounding tissues. Thereafter, the bearing element 5 is rotated in a counterclockwise oblique direction to align a position where the bearing element is combined with the tibial element 3. The present disclosure includes the recessed groove 3323 in the rim 332 unlike the prior art, thereby preventing the lower portion 53 of the bearing element from interfering with the rim 332 in the process of aligning the coupling position. Therefore, by solving the problem in that the lower portion 53 of the bearing element in the prior art climbs the rim 332 and the position is aligned in an excited state, so that, even when the bearing element 5 is inserted in the oblique direction, there is an effect that the bearing element 5 and the tibia element 3 can be easily coupled to each other at the correct position. Such a coupling relationship between the protruding portion 551 and a recessed portion 5323 can be seen in FIG. 12.

The detailed description above is illustrative of the present disclosure. In addition, the above description shows and describes preferred embodiments of the present disclosure, and the present disclosure can be used in various other combinations, modifications and environments. That is, changes or modifications may be made within the scope of the concept of the present disclosure disclosed in the present specification, the scope equivalent to the disclosed contents, and/or the skill or knowledge of the art. The above-described embodiments describe the best state for implementing the technical idea of the present disclosure, and various changes required in the specific application fields and uses of the present disclosure are possible. Therefore, the detailed description of the present disclosure is not intended to limit the present disclosure to the disclosed embodiment. In addition, the appended claims should be construed as including other embodiments.

## Claims

1. An artificial knee joint comprising:
a tibial element configured to be implanted at a proximal end of the tibia; and
a bearing element configured to be coupled to the tibial element,
wherein the tibial element includes an interference preventing portion capable of preventing interference with the bearing element when the bearing element is inserted in an oblique direction.

2. The artificial knee joint of claim 1, wherein the tibial element includes a base plate coupled to the bearing element, and the interference preventing portion is formed on the base plate.

3. The artificial knee joint of claim 2, wherein the interference preventing portion is formed on an outer periphery of the tibial element.

4. The artificial knee joint of claim 3, wherein the interference preventing portion is a recessed groove formed by being recessed at a predetermined position of the outer periphery of the tibial element.

5. The artificial knee joint of claim 4, wherein the base plate includes a rim protruding along an outer periphery thereof, and the recessed groove is formed in the rim.

6. The artificial knee joint of any one of claims 2 to 5, wherein the interference preventing portion is formed symmetrically with respect to line A-P.

7. The artificial knee joint of any one of claims 2 to 5, wherein the interference preventing portion includes one end and the other end, and one end is located at a posterior side and the other end is located at an anterior side with respect to line M-L.

8. The artificial knee joint of any one of claims 1 to 5, wherein the bearing element includes a protrusion coupled to the interference preventing portion.

9. The artificial knee joint of any one of claim 8, wherein the protrusion has a shape complementary to the interference preventing portion.
